# EUROPEAN PATENT APPLICATION

(11) **EP 2 108 325 A1**
(43) Date of publication of application: **14.10.2009**
(21) Application number: 09005160.8
(22) Date of filing: 08.04.2009
(51) Int. Cl.: A61B 18/12, A61B 18/14, H01R 13/46

(54) **ARC generation in a fluid medium**

(30) Priority: 08.04.2008 US 43240 P; 20.03.2009 US 407896
(71) Applicant: Tyco Healthcare Group, LP, North Haven CT 06473 (US)
(72) Inventor: Craig, Jason L., Loveland CO 80537 (US)
(74) Representative: HOFFMANN EITLE

(57) **Abstract**

An electrosurgical generator is disclosed. The generator includes a radio frequency output stage configured to generate a frequency waveform to an active electrode of an electrosurgical instrument when the active electrode is disposed in a fluid medium. The generator also includes a sensor circuit configured to measure tissue impedance and a controller configured to increase power of the radio frequency waveform to a predetermined electrical arcing level in response to the tissue impedance being within an open circuit impedance range and tissue contact impedance range. The controller is further configured to lower the power of the radio frequency waveform to a lower level when the tissue impedance is outside the open circuit impedance range and the tissue contact impedance range.

## Description

### CROSS-REFERENCE TO RELATED APPLICATIONS

This application claims the benefit of priority to U.S. Provisional Application Serial No. 61/043,240 entitled "ARC GENERATION IN A FLUID MEDIUM" filed April 8, 2008 by Jason L. Craig, which is incorporated by reference herein.

### BACKGROUND

### Technical Field

The present disclosure relates to electrosurgical apparatuses, systems and methods. More particularly, the present disclosure is directed to an electrosurgical generator configured to generate arcs in a liquid medium wherein the output of the generator is adjusted by a feedback control loop.

### Background of Related Art

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current to a surgical site to cut, ablate, coagulate or seal tissue. In monopolar electrosurgery, an active electrode delivers radio frequency energy from the electrosurgical generator to the tissue and a return electrode carries the current back to the generator. In monopolar electrosurgery, the active electrode is typically part of the surgical instrument held by the surgeon and applied to the tissue to be treated. A patient return electrode is placed remotely from the active electrode to carry the current back to the generator.

Ablation is most commonly a monopolar procedure that is particularly useful in the field of cancer treatment, where one or more RF ablation needle electrodes (usually having elongated cylindrical geometry) are inserted into a living body and placed in the tumor region of an affected organ. A typical form of such needle electrodes incorporates an insulated sheath from which an exposed (uninsulated) tip extends. When RF energy is provided between the return electrode and the inserted ablation electrode, RF current flows from the needle electrode through the body. Typically, the current density is very high near the tip of the needle electrode, which tends to heat and destroy surrounding issue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned immediately adjacent the electrodes. When the electrodes are sufficiently separated from one another, the electrical circuit is open and thus inadvertent contact with body tissue with either of the separated electrodes does not cause current to flow.

Certain types of electrosurgical procedures are performed in a liquid medium, such as transurethral resections of the prostate ("TURP") which are generally performed in a 1.5% sorbitol distension medium. Electrosurgical resection of tissue requires the production of arcs between the active electrode and the tissue. However, it is particularly difficult to resect tissue efficiently in particular environments due to inherent problems in producing electrical arcing. More specifically, a liquid with a low impedance path tends to inhibit the production of electrical arcs. Therefore it is desirable to provide for an electrosurgical generator configured to generate electrical arcs in a low impedance medium.

### SUMMARY

According to one aspect of the present disclosure an electrosurgical generator is disclosed. The generator includes a radio frequency output stage configured to generate a frequency waveform to an active electrode of an electrosurgical instrument when the active electrode is disposed in a fluid medium. The generator also includes a sensor circuit configured to measure tissue impedance and a controller configured to increase power of the radio frequency waveform to a predetermined electrical arcing level in response to the tissue impedance being within an open circuit impedance range and tissue contact impedance range. The controller is further configured to lower the power of the radio frequency waveform to a lower level when the tissue impedance is outside the open circuit impedance range and the tissue contact impedance range.

A method for operating an electrosurgical generator is also contemplated by the present disclosure. The method includes the steps of providing an electrosurgical instrument having an active electrode, submerging the active electrode into a fluid medium and generating a radio frequency waveform to the active electrode of the electrosurgical instrument. The method also includes the steps of measuring tissue impedance, increasing power of the radio frequency waveform to a predetermined electrical arcing level in response to the tissue impedance being within an open circuit impedance range and tissue contact impedance range to commence electrical arcing. The method further includes the step of lowering the power of the radio frequency waveform to a lower level to maintain arcing after initialization of the electrical arc.

According to another aspect of the present disclosure an electrosurgical system is disclosed. The electrosurgical system includes a generator having a radio frequency output stage configured to generate a radio frequency waveform and a sensor circuit configured to measure tissue impedance. The generator also includes a controller configured to increase power of the radio frequency waveform to a predetermined electrical arcing level in response to the tissue impedance being within an open circuit impedance range and tissue contact impedance range, the controller being further configured to lower the power of the radio frequency waveform to a lower level when the tissue impedance is outside the open circuit impedance range and the tissue contact impedance range. The system also includes an electrosurgical instrument coupled to the radio frequency output stage. The electrosurgical instrument includes an active electrode being disposed in a fluid medium and adapted to apply the radio frequency therein, the electrosurgical instrument further including an identifying element disposed on the electrosurgical instrument, the identifying element identifying the electrosurgical instrument to the generator such that the generator automatically programs preset electrosurgical parameters associated with the predetermined electrical arcing level, open circuit impedance range, tissue contact impedance range, and the lower level.

### BRIEF DESCRIPTION OF THE DRAWINGS

Various embodiments of the present disclosure are described herein with reference to the drawings wherein:
Figs. 1A-1B are schematic block diagrams of an electrosurgical system according to the present disclosure;
Fig. 2 is a schematic block diagram of a generator according to one embodiment of the present disclosure; and
Fig. 3 is a flow chart illustrating a method for controlling arc generation in a fluid medium according to the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

The generator according to the present disclosure can perform monopolar and bipolar electrosurgical procedures, including ablation, resection and/or vessel sealing procedures. The generator may include a plurality of outputs for interfacing with various electrosurgical instruments (e.g., a monopolar active electrode, return electrode, bipolar electrosurgical forceps, footswitch, etc.). Further, the generator includes electronic circuitry configured for generating radio frequency power specifically suited for various electrosurgical modes (e.g., cutting, blending, division, etc.) and procedures (e.g., monopolar, bipolar, vessel sealing).

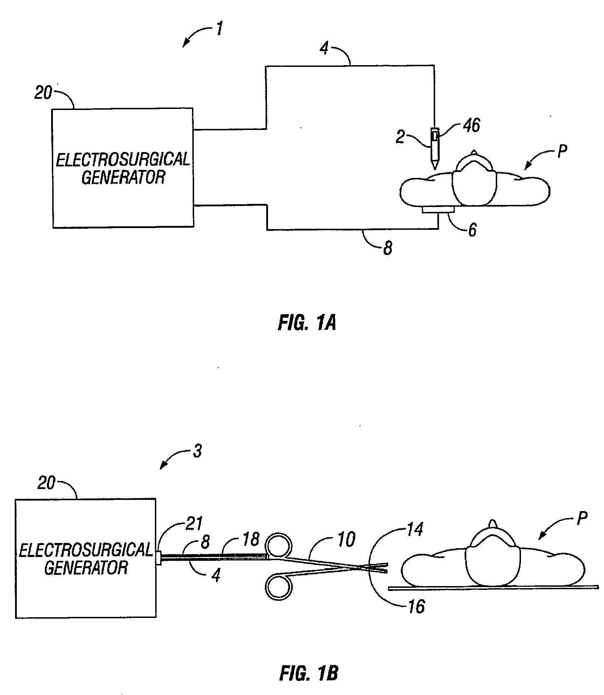

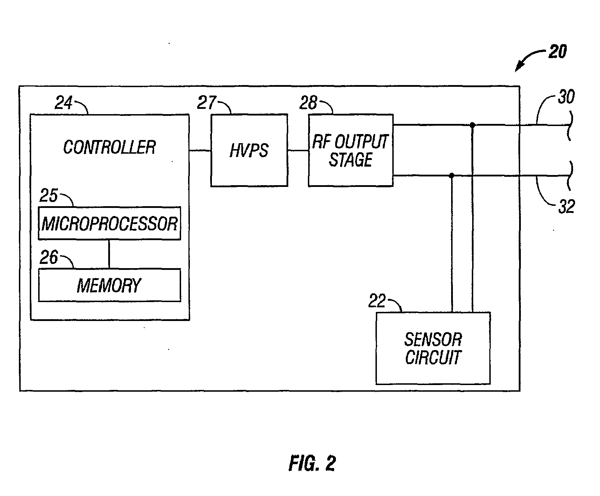

Fig. 1A is a schematic illustration of a monopolar electrosurgical system 1 according to one embodiment of the present disclosure. The system 1 includes an electrosurgical instrument 2 having one or more electrodes for treating tissue of a patient P. The instrument 2 is a monopolar type instrument including one or more active electrodes (e.g., electrosurgical cutting probe, ablation electrode(s), etc.). Electrosurgical RF energy is supplied to the instrument 2 by a generator 20 via a supply line 4, which is connected to an active terminal 30 (Fig. 2) of the generator 20, allowing the instrument 2 to coagulate, resect, ablate and/or otherwise treat tissue. The energy is returned to the generator 20 through a return electrode 6 via a return line 8 at a return terminal 32 (Fig. 2) of the generator 20. The active terminal 30 and the return terminal 32 are connectors configured to interface with plugs (not explicitly shown) of the instrument 2 and the return electrode 6, which are disposed at the ends of the supply line 4 and the return line 8, respectively.

The system 1 may include a plurality of return electrodes 6 that are arranged to minimize the chances of tissue damage by maximizing the overall contact area with the patient P. In addition, the generator 20 and the return electrode 6 may be configured for monitoring so-called "tissue-to-patient" contact to insure that sufficient contact exists therebetween to further minimize chances of tissue damage.

Fig. 1B is a schematic illustration of a bipolar electrosurgical system 3 according to the present disclosure. The system 3 includes a bipolar electrosurgical forceps 10 having one or more electrodes for treating tissue of a patient P. The electrosurgical forceps 10 include opposing jaw members having an active electrode 14 and a return electrode 16, respectively, disposed therein. The active electrode 14 and the return electrode 16 are connected to the generator 20 through cable 18, which includes the supply and return lines 4, 8 coupled to the active and return terminals 30, 32, respectively (Fig. 2). The electrosurgical forceps 10 is coupled to the generator 20 at a connector 21 having connections to the active and return terminals 30 and 32 (e.g., pins) via a plug disposed at the end of the cable 18, wherein the plug includes contacts from the supply and return lines 4, 8.

The generator 20 includes suitable input controls (e.g., buttons, activators, switches, touch screen, etc.) for controlling the generator 20. In addition, the generator 20 may include one or more display screens for providing the user with variety of output information (e.g., intensity settings, treatment complete indicators, etc.). The controls allow the user to adjust power of the RF energy, waveform, as well as the level of maximum arc energy allowed which varies depending on desired tissue effects and other parameters to achieve the desired waveform suitable for a particular task (e.g., coagulating, tissue sealing, intensity setting, etc.). The instrument 2 may also include a plurality of input controls (not explicitly shown) that may be redundant with certain input controls of the generator 20. Placing the input controls at the instrument 2 allows for easier and faster modification of RF energy parameters during the surgical procedure without requiring interaction with the generator 20.

Fig. 2 shows a schematic block diagram of the generator 20 having a controller 24, a high voltage DC power supply 27 ("HVPS") and an RF output stage 28. The HVPS 27 is connected to a conventional AC source (e.g., electrical wall outlet) and provides high voltage DC power to an RF output stage 28, which then converts high voltage DC power into RF energy and delivers the RF energy to the active terminal 30. The energy is returned thereto via the return terminal 32.

In particular, the RF output stage 28 generates either continuous or pulsed sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The radio frequency waveforms include a current and a voltage waveform. The present disclosure provides for a system and method which monitors and compares the voltage and current waveform to detect discrepancies between the waveform on a time scale substantially equal to one-half of the radio frequency cycle of the waveform.

The generator 20 may include a plurality of connectors to accommodate various types of electrosurgical instruments (e.g., instrument 2, electrosurgical forceps 10, etc.). Further, the generator 20 may operate in monopolar or bipolar modes by including a switching mechanism (e.g., relays) to switch the supply of RF energy between the connectors, such that, for instance, when the instrument 2 is connected to the generator 20, only the monopolar plug receives RF energy.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port (not shown) that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor or analog circuitry (e.g., control circuit) adapted to perform the calculations discussed herein.

The generator 20 may implement a closed and/or open loop control schemes which include a sensor circuit 22 having a plurality of sensors measuring a variety of tissue and energy properties (e.g., tissue impedance, tissue temperature, output current and/or voltage, etc.), and providing feedback to the controller 24. A current sensor (not explicitly shown) can be disposed at either the active or return current path (or both) and voltage can be sensed at the active electrode(s). The controller 24 compares voltage and current waveforms to identify arc events, the duration thereof and total energy of the arc event. The controller 24 then transmits appropriate signals to the HVPS 27 and/or RF output stage 28, which then adjust DC and/or RF power supply, respectively, by using a maximum allowable arc energy which varies according to the selected mode. The controller 24 also receives input signals from the input controls of the generator 20 or the instrument 2. The controller 24 utilizes the input signals to adjust power output by the generator 20 and/or performs other control functions thereon.

The system 1 is particularly suitable for tissue resection in pure cut, blend, fulguration and desiccation electrosurgical modes. These modes are useful for resection procedures since they are particularly designed to produce arcing. Conversely, other electrosurgical modes, e.g., coagulation mode, do not generate arcing and instead simply heat tissue. During a resection procedure, the instrument 2 is submerged in the fluid medium at the tissue site and the generator 20 is activated in a suitable mode to produce arcing at the tissue site. In one embodiment, the system 3 may also be used in a cutting mode, with both the active electrode 14 and the return electrode 16 acting as an active electrode and one or more return electrode pads acting as a neutral electrode.

The generator 20 produces an initial so-called "working" arc for tissue resection and/or desiccation in a fluid medium by utilizing higher initial power. Once the arc is established, a lower power is used to maintain arcing. The generator 20 incorporates a feedback loop for monitoring current, voltage and/or impedance at the tissue site to regulate arcing.

Initially, the generator 20 monitors the impedance for a specific value associated with an open circuit activation of the instrument 2 in the fluid medium. Once a different impedance value is detected, which is indicative of tissue contact, output power of the generator 20 is increased to a predetermined electrical arcing level which is configured to generate a working arc. Once the working arc is established, the impedance level increases. The increase in impedance is detected and the power is decreased to a lower level to maintain arcing. In one embodiment, every time the open circuit impedance is detected, which denotes loss of the arc, the generator 20 boosts the power to the predetermined electrical arcing level to reinitialize the arc.

In another embodiment, the impedance at the target tissue site is monitored to determine whether tissue contact has occurred between the instrument 2 and the tissue. Once the determination is made that tissue contact has occurred, power is increased to the predetermined electrical arcing level to create a working arc. Conversely, once the generator 20 detects a change in impedance indicative of an open circuit and premature termination of the arc, the generator 20 terminates the output to prevent simply heating tissue without cutting. The user then reactivates the generator 20 which outputs power at the predetermined electrical arcing level to reinitialize the arc.

In a further embodiment, as shown in Fig. 1, the instrument 2 includes an identifying element 46, which can be a barcode, a radio frequency identification ("RFID") tag, one or more resistors which have a unique resistance corresponding to an identifying code, or a magnetic identifier such as such as gray coded magnets and/or ferrous nodes incorporating predetermined unique magnetic patterns configured to be read by magnetic sensor (e.g., ferromagnetic sensor, Hall effect sensor, etc.). The identifying element 46 stores an identifying code associated with the instrument 2 which identifies the instrument 2 as suitable for the fluid medium procedure (e.g., TURP).

The generator 20 includes an identifying element reader (e.g., barcode reader, RFID interrogator, ferromagnetic sensor, Hall effect sensor, resistance sensors, etc.) configured to interface with the identifying element 46. Once the generator 20 obtains the identifying code, the generator 20 automatically configures operating parameters, such as the predetermined electrical arcing level, impedance ranges (e.g., open circuit impedance, tissue contact impedance, etc.), current and voltage levels which are used to maintain arcing during the resection procedure.

The open circuit impedance range may be approximately equal to or above the maximum impedance associated with desiccated tissue, such range being from about 5000 Ohms to about infinite impedance. The tissue contact impedance range may be from about 20 Ohms to about 5000 Ohms, such that a short-circuit does not fall within the tissue contact range. Those skilled in the art will appreciate that the recited impedance values very based on the frequency of the energy being supplied and the medium in which the procedure is being performed.

In addition to preprogramming operating parameters into the generator via the identifying element 46, the generator 20 can also be programming to utilize a custom power curve configured to be used in conjunction with the operating parameters of the identifying element 46. The custom power curve incorporates power adjustments made in response to the impedance being within open circuit and/or tissue contact impedance ranges. Thus, the power curve is adapted to adjust the output of the generator 20 to the predetermined electrical arcing level when impedance is within the open circuit impedance range and to lower the output once arcing is established.

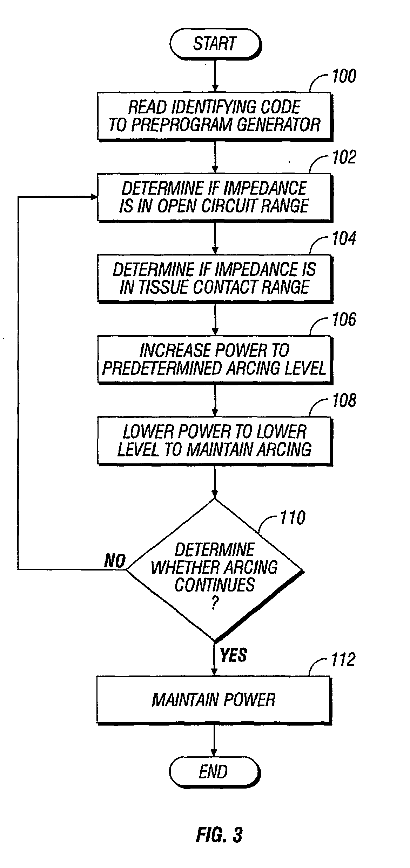

Fig. 3 illustrates a method for controlling arcing in a fluid medium. In step 100, the identifying code from the identifying element 46 is read by the generator 20. The generator 20 is then preset based on the identifying code. More specifically, the generator 20 stores within the memory 26 various operating parameters and power curves associated with the identifying code. Once the code is read, the generator 20 loads impedance ranges, such as open circuit impedance range, a tissue contact impedance range, and power levels, such as a predetermined electrical arcing level and a lower level. In addition, a power curve is also loaded which adjusts the output of the generator 20 to the desired power levels. The impedance ranges and power levels are determined through empirical levels and are preloaded into the generator 20.

In step 102, the instrument 2 is brought into the fluid medium and an initial impedance measurement is taken. The generator 20 determines whether the initial impedance value is within the open circuit impedance range, which is associated with an open circuit activation of the instrument 2 in the fluid medium. In step 104, the generator 20 measures tissue impedance again to determine whether the impedance is within a tissue contact impedance range. The tissue contact impedance range is defined as a change in impedance in reference to the impedance measurement taken in step 102.

Once it is determined that the impedance is within the tissue contact impedance range, the generator 20 commences power application. In step 106, power is increased to the predetermined electrical arcing level to provide arcing between the instrument 2 and the tissue to facilitate resectioning tissue.

Once arcing is established, impedance is continually monitored to determine whether there is a change in impedance. If there is a change in impedance, in step 108, the power is lowered to the lower level to maintain arcing. In step 110, the generator 20 determines continuous arcing by comparing the measured impedance to the open circuit and tissue contact impedance ranges. If arcing continues, then the generator 20 maintains power. If, in contrast, the impedance is within those ranges, the generator reinitializes arcing by boosting power up to the predetermined electrical arcing level.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical generator, comprising:
a radio frequency output stage configured to generate at least one radio frequency waveform to an active electrode of an electrosurgical instrument when the active electrode is disposed in a fluid medium;
a sensor circuit configured to measure tissue impedance; and
a controller configured to increase power of the at least one radio frequency waveform to a predetermined electrical arcing level in response to the tissue impedance being within an open circuit impedance range and tissue contact impedance range, the controller being further configured to lower the power of the at least one radio frequency waveform to a lower level when the tissue impedance is outside the open circuit impedance range and the tissue contact impedance range.

2. The electrosurgical generator according to claim 1, wherein the electrosurgical generator includes an identifying element reader configured to interface with an identifying element disposed on the electrosurgical instrument, the identifying element identifying the electrosurgical instrument to the generator such that the generator automatically programs preset electrosurgical parameters associated with the predetermined electrical arcing level, open circuit impedance range, tissue contact impedance range, and the lower level.

3. The electrosurgical generator according to claim 2, wherein the identifying element is a barcode, a radio frequency identification tag, a resistor or a magnetic identifier.

4. The electrosurgical generator according to claim 1 2 or 3, wherein the controller is further configured to increase power of the at least one radio frequency waveform to the predetermined electrical arcing level in response to the tissue impedance returning to the open circuit impedance range and the tissue contact impedance range to reinitialize arcing.

5. The electrosurgical generator according to claim 1, 2 or 3, wherein the controller is further configured to terminate output of the radio frequency output stage in response to the tissue impedance returning to the open circuit impedance range and the tissue contact impedance range.

6. The electrosurgical generator according to any one of the preceding claims, wherein the open circuit impedance range is from about 5000 Ohms to about infinite impedance and the tissue contact impedance range is from about 20 Ohms to about 5000 Ohms.

7. A method of preparing an electrosurgical generator for use, comprising the steps of:
providing an electrosurgical instrument having at least one electrode;
submerging the at least one active electrode into a fluid medium
generating at least one radio frequency waveform to the at least one active electrode of the electrosurgical instrument;
measuring tissue impedance;
increasing power of the at least one radio frequency waveform to a predetermined electrical arcing level in response to the tissue impedance being within an open circuit impedance range and tissue contact impedance range to commence electrical arcing; and
lowering the power of the at least one radio frequency waveform to a lower level to maintain arcing after initialization of the electrical arc.

8. The method according to claim 7, further comprising the step of:
interfacing with an identifying element disposed on the electrosurgical instrument to identify the electrosurgical instrument to the generator such that the generator automatically programs preset electrosurgical parameters associated with the predetermined electrical arcing level, open circuit impedance range, tissue contact impedance range, and the lower level.

9. The method according to claim 7 or 8, further comprising the step of:
increasing power of the at least one radio frequency waveform to the predetermined electrical arcing level in response to the tissue impedance returning to the open circuit impedance range and the tissue contact impedance range to reinitialize arcing.

10. The method according to claim 7, 8 or 9, further comprising the step of:
terminating output of the radio frequency output stage in response to the tissue impedance returning to the open circuit impedance range and the tissue contact impedance range.

11. An electrosurgical system comprising:
an electrosurgical generator comprising:
a radio frequency output stage configured to generate at least one radio frequency waveform;
a sensor circuit configured to measure tissue impedance; and
a controller configured to increase power of the at least one radio frequency waveform to a predetermined electrical arcing level in response to the tissue impedance being within an open circuit impedance range and tissue contact impedance range, the controller being further configured to lower the power of the at least one radio frequency waveform to a lower level when the tissue impedance is outside the open circuit impedance range and the tissue contact impedance range;
and
an electrosurgical instrument coupled to the radio frequency output stage, the electrosurgical instrument including at least one active electrode being disposed in a fluid medium and adapted to apply the at least one radio frequency therein, the electrosurgical instrument further including an identifying element disposed on the electrosurgical instrument, the identifying element identifying the electrosurgical instrument to the generator such that the generator automatically programs preset electrosurgical parameters associated with the predetermined electrical arcing level, open circuit impedance range, tissue contact impedance range, and the lower level.

12. The electrosurgical system according to claim 11, wherein the controller is further configured to increase power of the at least one radio frequency waveform to the predetermined electrical arcing level in response to the tissue impedance returning to the open circuit impedance range and the tissue contact impedance range to reinitialize arcing.

13. The electrosurgical system according to claim 11 or 12, wherein the controller is further configured to terminate output of the radio frequency output stage in response to the tissue impedance returning to the open circuit impedance range and the tissue contact impedance range.

14. The electrosurgical system according to any one of claims 11 to 13, wherein the open circuit impedance range is from about 5000 Ohms to about infinite impedance and the tissue contact impedance range is from about 20 Ohms to about 5000 Ohms.

15. A method of preparing an electrosurgical instrument for use comprising identifying the instrument to an electrosurgical generator with an identifying element on the instrument, such that the generator automatically programs preset electrosurgical parameters associated with a predetermined electrical arcing level, open circuit impedance range, tissue contact impedance range and a lower level that maintains arcing after initialization of the electrical arc.
